# FASCICULE DE BREVET EUROPEEN

(11) **EP 0 515 274 B1**
(45) Date de publication et mention de la délivrance du brevet: **17.01.1996**
(21) Numéro de dépôt: 92401390.7
(22) Date de dépôt: 21.05.1992
(51) Int. Cl.: A61C 8/00, A61B 17/16

(54) **Perfectionnements aux forets hélicoidaux pour la chirurgie osseuse, en particulier pour la chirurgie dentaire**
Verbesserungen an Spiralbohrern für die Knochenchirurgie, insbesondere für die Zahnchirurgie
Improvements to helical drills for bone surgery, in particular for dental surgery

(30) Priorité: 21.05.1991 FR 9106093
(43) Date de publication de la demande: 25.11.1992
(73) Titulaire: PELTIER, Patrick, F-92500 Rueil-Malmaison (FR); PELTIER, Guy, F-92500 Rueil-Malmaison (FR)
(72) Inventeur: PELTIER, Patrick, F-92500 Rueil-Malmaison (FR); PELTIER, Guy, F-92500 Rueil-Malmaison (FR)
(74) Mandataire: Bernasconi, Jean

(56) Documents cités:
- DE-A- 3 800 482
- DE-A- 4 017 038
- GB-A- 2 119 258
- US-A- 4 474 556

## Description

L'invention est relative aux forets hélicoïdaux pour la chirurgie osseuse et elle concerne plus particulièrement, mais non exclusivement, les forets pour la chirurgie dentaire, c'est-à-dire les forets destinés à percer ou à aléser, dans le maxillaire humain, des logements pour implants dentaires.

On sait que ces forets sont constitués d'un corps cylindrique dans lequel sont creusées au moins deux, en général de deux à quatre, rainures hélicoïdales qui délimitent autant de lèvres de coupe hélicoïdales. Ce corps est prolongé par une queue destinée à être emmanchée dans un porte-foret ou mandrin qui, en fonctionnement, communique au foret son mouvement de rotation.

On sait également que, dans la chirurgie osseuse, le praticien doit arrêter le foret aussi exactement que possible à la profondeur voulue qui est en général déterminée au préalable par radiographie. En particulier, dans la chirurgie dentaire prothétique, le logement ou trou à pratiquer dans le maxillaire doit avoir une profondeur suffisamment grande pour assurer, à l'implant cylindrique (en titane, vissé ou lisse) à y insérer, un ancrage robuste mais limitée à une valeur qui lui interdit de déboucher dans le sinus maxillaire. En général, le praticien ne dispose pas de repère lui permettant d'atteindre avec sécurité en une seule fois la profondeur voulue et il est donc obligé de s'y reprendre à plusieurs fois avec le même foret, en mesurant entre chaque passe la profondeur d'enfoncement de ce foret et en la comparant alors à la valeur voulue. Pour faciliter le travail du praticien, certains forets présentent sur leurs lèvres de coupe, à des distances axiales régulières, des repères constitués d'encoches en V de faible profondeur radiale (0,1 mm environ) mais il est difficile au praticien de ne pas confondre deux encoches successives au cours de son travail, surtout lorsqu'il doit opérer sur la partie postérieure de la mâchoire d'un patient, ce qui prive ces encoches de la sécurité nécessaire.

Des inconvénients analogues se présentent dans la chirurgie osseuse générale, lorsqu'il s'agit par exemple d'implanter une broche. Pour éliminer ces inconvénients, il a été proposé (DE-A-3.800.482), formant le préambule de la revendication 1 de munir le foret de repères d'enfoncement qui comprennent des gorges circonférentielles de profil sensiblement en arc de cercle, réparties à des distances axiales prédéterminées, et qui agissent par arrivée en contact avec l'os à traiter mais, dans cette construction connue, un manchon en tissu est disposé autour du foret et est immobilisé par introduction de billes dans les gorges qui sont ménagées dans la queue d'entraînement et c'est l'arrivée en contact de l'extrémité inférieure de ce manchon avec l'os à traiter qui signale que la profondeur voulue d'enfoncement est atteinte. Ce foret connu a pour inconvénients d'être compliqué et coûteux, imprécis et difficile à stériliser.

L'invention a pour but de remédier aux susdits inconvénients en dotant les forets de repères réglables et bien visibles dans les conditions opératoires les plus délicates, et ceci sans modifier de façon notable la forme usuelle des forets et par conséquent le procédé de fabrication de ceux-ci ni sans augmenter sensiblement leur prix de revient.

A cet effet, l'invention a pour objet un foret hélicoïdal pour la chirurgie osseuse, notamment pour la chirurgie dentaire, constitué d'un corps cylindrique dans lequel sont creusées au moins deux rainures hélicoïdales qui délimitent autant de lèvres de coupe hélicoïdales, ce corps étant prolongé par une queue d'entraînement et le foret étant muni de repères d'enfoncement qui comprennent des gorges circonférentielles de profil sensiblement en arc de cercle, réparties à des distances axiales déterminées, et qui agissent par arrivée en contact avec l'os ou la dent à traiter, caractérisé en ce que les gorges sont ménagées dans le corps cylindrique et sont agencées pour recevoir une bague élastique qui a un profil adapté à celui des gorges et une forme plus ou moins torique et qui est susceptible d'être placée à volonté dans au moins l'une ou l'autre de ces gorges avant mise en action du foret et de s'y maintenir jusqu'à la fin de cette action, laquelle fin est déterminée par l'arrivée en contact de la bague avec l'os ou la dent à traiter.

Chaque gorge circonférentielle ainsi ménagée dans le corps du foret est bien entendu divisée en tronçons par les rainures hélicoïdales. En d'autres termes, elle n'intéresse que les lèvres de coupe mais constitue, par l'ensemble de ses tronçons successifs, un logement capable de retenir la bague élastique qui y serait placée.

S'agissant de forets pour la chirurgie dentaire dont le diamètre extérieur du corps est en général au plus égal à 4 mm, on donne de préférence à la bague élastique un diamètre extérieur, en coupe transversale, de l'ordre de 1 mm et aux gorges annulaires des distances mutuelles régulières comprises entre 1 et 2 mm, par exemple égales à 2 mm. Selon un mode de réalisation préféré, ces gorges sont situées respectivement à 8, 10, 12, 14, 16, 18 et 20 mm de l'extrémité du foret opposée à la queue.

La bague élastique est de préférence faite de caoutchouc ou matière plastique dont la composition, homologuée dans l'application envisagée, est susceptible de résister aux conditions d'aseptisation ainsi qu'aux échauffements dus au travail du foret et à l'arrivée en contact éventuelle avec l'os à forer, tel qu'un maxillaire. La bague peut néanmoins être aussi faite d'acier inoxydable à ressort et être constituée par exemple d'un ressort hélicoïdal refermé sur lui-même en anneau, par vissage de l'une de ses extrémités dans l'autre.

L'invention va être maintenant décrite plus en détail à l'aide des dessins annexés.

La figure 1 de ces dessins représente, en élévation, un foret établi conformément à l'invention sur lequel sont placées des bagues élastiques de trois types différents, une seule bague devant bien entendu être mise en place dans les conditions effectives d'utilisation.

La figure 2 représente un foret de perçage, en coupe transversale selon la ligne II-II de la figure 1.

Les figures 3 et 4 représentent, par des vues en coupe transversale analogues à celle de la figure 2, des forets aléseurs à trois et quatre lèvres de coupe respectivement.

La figure 5 représente, en coupe transversale par son plan de symétrie, l'une des bagues de la figure 1.

La figure 6 représente une variante du mode de réalisation de la figure 1.

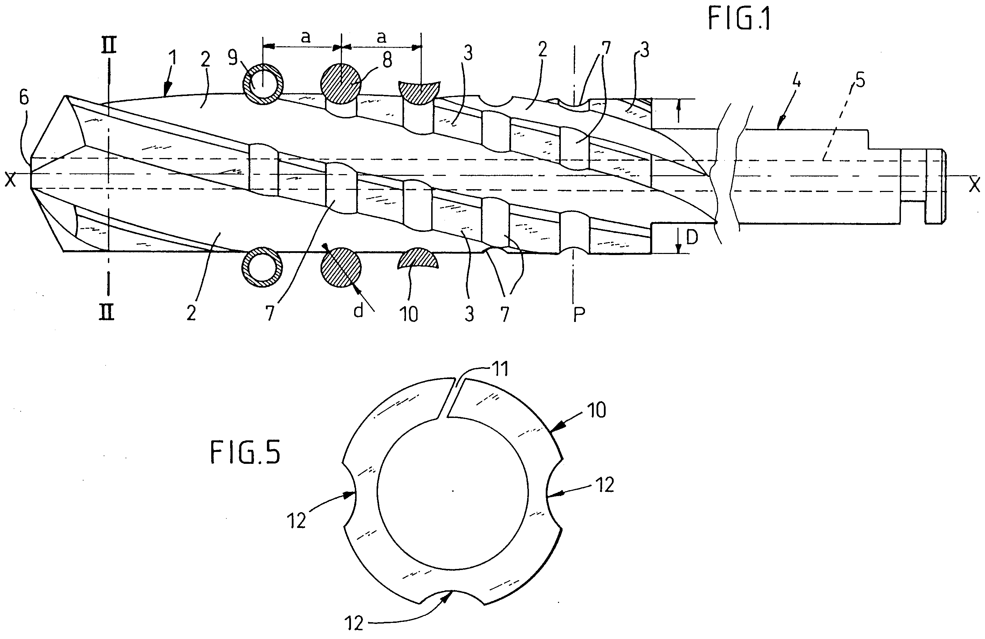

Comme le montre schématiquement la figure 1, un foret pour la chirurgie dentaire est constitué d'un corps cylindrique 1 dans lequel sont creusées au moins deux rainures hélicoïdales 2 qui délimitent autant de lèvres de coupe hélicoïdales 3. Le corps 1 est prolongé par une queue d'entraînement 4. Le corps 1 et la queue 4 sont en général munis d'un canal d'irrigation longitudinal 5.

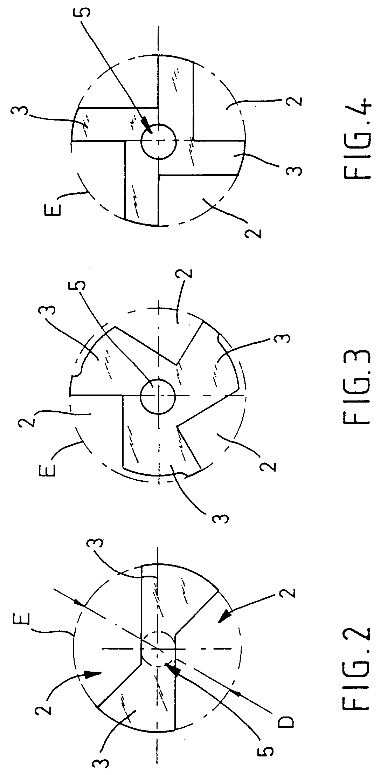

Il peut s'agir d'un foret de perçage, à deux rainures 2 et deux lèvres de coupe 3, comme représenté à la figure 2, auquel cas le canal d'irrigation 5 est borgne mais débouche par des passages radiaux. Il peut également s'agir d'un foret d'alésage à trois rainures 2 et trois lèvres de coupe 3, comme représenté à la figure 3, auquel cas le canal d'irrigation 5 débouche à l'extrémité libre 6 du foret, c'est-à-dire à l'extrémité opposée à la queue 4. Le foret d'alésage de la figure 4 diffère de celui de la figure 3 essentiellement par le nombre des rainures 2 et des lèvres de coupe 3 qui est ici égal à quatre. Dans ce qui précède, lorsqu'on a qualifié de "cylindrique" le corps 1, on a voulu dire un corps dont l'enveloppe géométrique E est cylindrique, comme indiqué en trait mixte à chacune des figures 2 à 4.

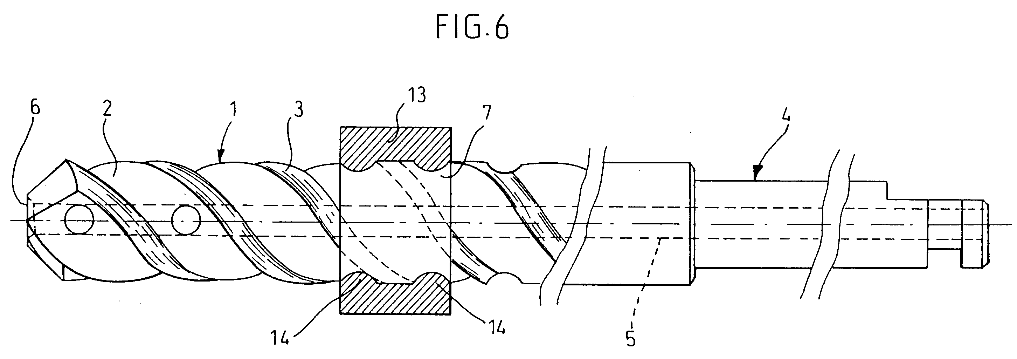

Cela étant, conformément à l'invention et comme représenté à la figure 1, le corps 1 est muni de gorges circonférentielles 7, de profil sensiblement en arc de cercle, admettant chacune un plan de symétrie, tel que P, perpendiculaire à l'axe longitudinal X-X du foret, axe qui constitue l'axe de rotation du foret et l'axe de révolution de l'enveloppe E. De plus, on associe à chaque foret une bague élastique, telle que 8, 9 ou 10, qui a un profil adapté à celui des gorges 7 et une forme plus ou moins torique et qui est susceptible d'être placée à volonté dans l'une ou l'autre de ces gorges 7. Ainsi qu'il a été exposé ci-dessus, on voit à la figure 1 que chaque gorge 7 est divisée en tronçons successifs par les rainures 2 mais constitue un logement sûr pour une bague élastique ayant la forme d'un anneau continu, voire d'un anneau présentant une étroite fente radiale, telle que celle représentée en 11 à la figure 5.

Le corps 1 du foret pour chirurgie dentaire représenté à la figure 1 possède un diamètre extérieur D (ou diamètre de la directrice de son enveloppe E, voir la figure 2) qui est en général au plus égal à 4 mm. Dans ce cas, on donne de préférence à la bague élastique telle que 8 un diamètre extérieur d, en coupe transversale, de l'ordre de 1 mm. Ceci signifie que le rayon de courbure de la section des gorges 7 par un plan passant par l'axe X-X est de l'ordre de 0,5 mm.

Les gorges 7 sont disposées à des distances mutuelles régulières "a" comprises entre 1 et 2 mm, de préférence égales à 2 mm. De préférence encore, et bien que la figure 1 représente un plus petit nombre de gorges 7, celles-ci sont avantageusement situées à 8, 10, 12, 14, 16, 18 et 20 mm de l'extrémité libre 6 du foret (distances mesurées à partir des plans de symétrie respectifs P définis ci-dessus).

La figure 1 montre en 8 une bague torique en caoutchouc ou matière plastique souple ; en 9 une bague constituée d'un ressort hélicoïdal dont les spires terminales sont vissées l'une dans l'autre et en 10 (voir aussi la figure 5) une bague tubulaire en acier inoxydable à ressort, munie de la susdite fente 11 et d'encoches 12 facilitant sa flexion.

Selon la variante de la figure 6, la bague 13, qui est caoutchouc ou matière plastique souple, possède deux bourrelets intérieurs continus 14, dont la distance axiale est égale à la distance "a" entre gorges 7, ce qui lui permet de s'engager dans deux gorges consécutives et améliore le maintien en place de la bague 13.

On obtient ainsi un foret pour la chirurgie dentaire dont le fonctionnement est le suivant.

De façon connue, on utilise d'abord le foret de perçage de la figure 2, dont le diamètre est en général compris entre 2,1 et 2,9 mm, après y avoir placé l'une des bagues telles que 8 à 10 dans celle de ses gorges 7 qui correspond à la longueur de l'implant à insérer dans l'os. On utilise ensuite le foret d'alésage de la figure 3, puis le cas échéant celui de la figure 4 (forets dont les diamètres respectifs sont compris entre celui du foret de perçage et 4 mm), après y avoir de même placé une bague dans l'une des gorges 7. Selon une variante (non représentée), il est possible de réaliser un foret combiné et de perçage et d'alésage, auquel cas il suffit, en partant du mode de réalisation de la figure 1, de raccourcir le corps 1 et de disposer, entre la queue 4 et le corps 1 ainsi raccourci, un corps de foret d'alésage de plus grand diamètre que ce corps 1, ce qui permet d'effectuer les opérations de perçage et d'alésage avec le même foret. Une fois ces opérations de perçage et d'alésage achevées, il est possible de visser ou d'insérer à force l'implant adéquat. Les forets devant être utilisés à ces opérations sont nettoyés et stérilisés avec leur bague préalablement mise en place, dans les appareils médicaux adaptés aux normes d'hygiène en vigueur.

De toute façon, l'élasticité intrinsèque de la bague choisie permet au praticien de la faire passer d'une gorge 7 (ou d'une paire de gorges 7, selon la variante de la figure 6) à l'autre, jusqu'à la gorge (ou paire de gorges) correspondant à la profondeur voulue de travail, mais la maintient ensuite dans cette dernière gorge (ou paire de gorges) pendant les opérations d'aseptisation, puis de perçage ou d'alésage. Une fois mise en place, la bague constitue un repère indéréglable et bien visible, quelles que soient la position angulaire instantanée et la vitesse de rotation du foret.

Bien que le foret conforme à l'invention ait été décrit à propos de son application à la chirurgie dentaire, il va de soi qu'il peut être aussi bien utilisé dans la chirurgie osseuse générale.

## Revendications

1. Foret hélicoïdal pour la chirurgie osseuse, notamment pour la chirurgie dentaire, constitué d'un corps cylindrique (1) dans lequel sont creusées au moins deux rainures hélicoïdales (2) qui délimitent autant de lèvres de coupe hélicoïdales (3), ce corps (1) étant prolongé par une queue d'entraînement (4) et le foret étant muni de repères d'enfoncement qui comprennent des gorges circonférentielles (7) de profil sensiblement en arc de cercle, réparties à des distances axiales déterminées, et qui agissent par arrivée en contact avec l'os ou la dent à traiter, caractérisé en ce que les gorges (7) sont ménagées dans le corps cylindrique (1) et sont agencées pour recevoir une bague élastique (8, 9, 10 ou 13) qui a un profil adapté à celui des gorges (7) et une forme plus ou moins torique et qui est susceptible d'être placée à volonté dans au moins l'une ou l'autre de ces gorges (7) avant mise en action du foret et de s'y maintenir jusqu'à la fin de cette action, déterminée par l'arrivée en contact de la bague (8, 9, 10 ou 13) avec l'os ou la dent à traiter.

2. Foret pour la chirurgie dentaire selon la revendication 1 dont le diamètre du corps (1) est au plus égal à 4 mm, caractérisé en ce que la bague élastique (8, 9 ou 10) a un diamètre extérieur, en coupe transversale, de l'ordre de 1 mm.

3. Foret selon la revendication 2, caractérisé en ce que les gorges annulaires (7) sont disposées à des distances mutuelles régulières comprises entre 1 et 2 mm.

4. Foret selon la revendication 3, caractérisé en ce que les gorges annulaires (7) sont disposées à des distances mutuelles de 2 mm.

5. Foret selon la revendication 4, caractérisé en ce que les gorges (7) sont situées respectivement à 8, 10, 12, 14, 16, 18 et 20 mm de l'extrémité (6) du foret opposée à la queue (4).

6. Foret selon l'une quelconque des revendications 1 à 5, caractérisé en ce que la bague (8) est faite de caoutchouc ou matière plastique dont la composition, homologuée dans l'application envisagée, est susceptible de résister aux conditions d'aseptisation ainsi qu'aux échauffements dus au travail du foret et à l'arrivée en contact éventuelle avec l'os à forer, tel qu'un maxillaire.

7. Foret selon l'une quelconque des revendications 1 à 5, caractérisé en ce que la bague (9) est faite d'acier inoxydable à ressort et constituée par exemple d'un ressort hélicoïdal refermé sur lui-même en anneau, par vissage de l'une de ses extrémités dans l'autre.

## Claims

1. Helicoidal drill for bone surgery, especially for dental surgery, formed by a cylindrical body (1) in which are hollowed out at least two helicoidal grooves (2) which delimit as many helicoidal cutting lips (3), the body (1) being extended by an entraining rear portion (4) and the drill being equipped with penetration markets which comprise circumferential recesses (7) distributed at predetermined axial distances and having a profile substantially in the shape of an arc of a circle and which act as a result of coming into contact with the bone or the tooth to be treated, characterized in that the recesses (7) are arranged in the cylindrical body (1) and are designed to receive a resilient ring (8, 9, 10 or 13) which has a profile adapted to that of the recesses (7) and a more or less toric shape and which can be placed as desired in at least one or other of the recesses (7) before the drill is set in operation and can be maintained there until the end of that operation, which is determined by the ring (8, 9, 10 or 13) coming into contact with the bone or the tooth to be treated.

2. Drill for dental surgery according to Claim 1, the diameter of the body (1) of which is at most 4 mm, characterised in that the resilient ring (8, 9 or 10) has an outside diameter, in cross-section, of the order of 1 mm.

3. Drill according to Claim 2, characterised in that the annular recesses (7) are arranged at regular mutual distances of between 1 and 2 mm.

4. Drill according to Claim 3, characterised in that the annular recesses (7) are arranged at mutual distances of 2 mm.

5. Drill according to Claim 4, characterised in that the recesses (7) are arranged, respectively, at 8, 10, 12, 14, 16, 18 and 20 mm from the end (6) of the drill which is opposite the rear portion (4).

6. Drill according to any one of Claims 1 to 5, characterised in that the ring (8) is produced from rubber or plastics material of which the composition, proven in the application envisaged, is able to withstand sterilisation conditions and also heating resulting from the operation of the drill and any abutment with the bone to be drilled, such as a maxilla.

7. Drill according to any one of Claims 1 to 5, characterised in that the ring (9) is produced from spring stainless steel and is formed, for example, by a helicoidal spring which is closed on itself to form a ring by screwing one of its ends into the other.

## Patentansprüche

1. Spiralbohrer für die Knochenchirurgie, insbesondere für die Zahnchirurgie, bestehend aus einem zylindrischen Körper (1), in dem mindestens zwei 5 Spiralnuten (2) ausgenommen sind, die genausoviele spiralförmige Schneidlippen (3) begrenzen, wobei dieser Körper (1) sich in einem Einsteckende (4) fortsetzt und der Bohrer mit verieften Markierungen versehen ist, die Rillen in Umfangsrichtung (7) mit im wesentlichen kreisbogenförmigem Profil umfassen, in bestimmten Abständen in Achsenrichtung verteilt sind und die wirksam werden, wenn sie mit dem zu behandelnden Knochen oder Zahn in Berührung kommen, dadurch gekennzeichnet, daß die Rillen (7) im zylindrischen Körper (1) angebracht und dafür ausgebildet sind, einen elastischen Ring (8, 9, 10 oder 13) aufzunehmen, der ein Profil aufweist, das dem der Rillen (7) angepaßt ist, und eine mehr oder weniger torische Form hat und der geeignet ist, vor Benutzung des Bohrers nach Wunsch in mindestens die eine oder andere dieser Rillen (7) eingelegt zu werden und dort bis zum Ende dieses Vorganges zu bleiben, das dadurch festgestellt wird, daß dieser Ring (8, 9, 10 oder 11) mit dem zu behandelnden Knochen oder Zahn in Berührung kommt.

2. Bohrer für die Zahnchirurgie nach Patentanspruch 1, bei dem der Durchmesser des Körpers (1) höchstens gleich 4 mm ist, dadurch gekennzeichnet, daß der elastische Ring (8, 9 oder 10) im Querschnitt einen Außendurchmesser in der Größenordnung von 1 mm aufweist.

3. Bohrer nach Patentanspruch 2, dadurch gekennzeichnet, daß die ringförmigen Rillen (7) in regelmäßigen Abständen zwischen 1 und 2 mm voneinander angeordnet sind.

4. Bohrer nach Patentanspruch 3, dadurch gekennzeichnet, daß die ringförmigen Rillen (7) in Abständen von 2 mm voneinander angeordnet sind.

5. Bohrer nach Patentanspruch 4, dadurch gekennzeichnet, daß die Rillen (7) in 8, 10, 12, 14, 16, 18 bzw. 20 mm Abstand von demjenigen Ende (6) des Bohrers angeordnet sind, das dem Einsteckende (4) gegenüberliegt.

6. Bohrer nach irgendeinem der Patentansprüche 1 bis 5, dadurch gekennzeichnet, daß der Ring (8) aus Gummi oder Kunststoff gefertigt ist, dessen Zusammensetzung, für die beabsichtigte Anwendung amtlich zugelassen, in der Lage ist, den Bedingungen der Sterilisierung ebenso, wie der Erwärmungen aufgrund der Arbeit des Bohrers und bei eventueller Berührung mit dem zu bohrenden Knochen, wie etwa einem Kiefer, zu widerstehen.

7. Bohrer nach irgendeinem der Patentansprüche 1 bis 5, dadurch gekennzeichnet, daß der Ring (9) aus rostfreiem Stahl mit Feder gefertigt ist und beispielsweise aus einer in sich dadurch ringförmig geschlossenen Spiralfeder besteht, daß eines ihrer Enden ins andere geschraubt ist.
